# EUROPEAN PATENT SPECIFICATION

(11) **EP 2 620 129 B1**
(45) Date of publication and mention of the grant of the patent: **02.12.2020**
(21) Application number: 13000062.3
(22) Date of filing: 08.01.2013
(51) Int. Cl.: A61F 2/95

(54) **The system for introducing and positioning of a stent, in particular an intestinal stent.**
System zum Einführen und Positionieren eines Stents, insbesondere eines intestinalen Stents.
Système d'introduction et de positionnement d'endoprothèse, en particulier une endoprothèse intestinale.

(30) Priority: 09.01.2012 PL 39776612
(43) Date of publication of application: 31.07.2013
(73) Proprietor: BALTON Spolka z ograniczona odpowiedzialnoscia, 00-496 Warszawa (PL)
(72) Inventor: Plowiecki, Emil, 00-496 Warszawa (PL); Hurkala, Leszek, 00-496 Warszawa (PL); Gwiazdowska-Nowotka, Beata, 00-496 Warszawa (PL)
(74) Representative: Orlinska, Dorota Irena

(56) References cited:
- WO-A2-01/51114
- US-A1- 2008 132 989
- US-A1- 2009 264 859
- US-B1- 6 344 045

## Description

Object of the invention is a system for introducing and positioning a stent, in particular an intestinal stent. The invention relates to the field of medical devices for precise introducing into, and, then, positioning of a stent in tubular canals of the body, such as blood vessels, the urinary system, bile ducts, the gastrointestinal tract, which because of various diseases, have undergone contraction or blocking that disturbs normal functioning of organism.

In general, intestinal stents and catheters for their introducing and positioning are known and at present used in medicine. These are e.g. nitinol stents, for example duodenal stents of several centimeters length having diameter of 20-24 mm.

The treatment method based on placing a stent within the gastrointestinal tract, as a less invasive method, is in particular recommended in neoplastic diseases, e.g. in case of colorectal carcinoma. In many cases such illnesses result in a decrease in intestine patency. In case of surgical treatment, numerous literature data indicate that patient mortality rate is of the order 5 to 30%. The surgical treatment is associated with a very high risk at elderly patients and in case of an advanced disease stage or in case of patients suffering from other diseases. As a less invasive alternative to the surgical treatment, stents are more and more recommended by physicians as a way for improving a quality of life of patients or a temporal postponement of a surgical treatment.

Intestinal stents may have different uses, and accordingly they are characterized by suitably modified construction.

From US patent application US2005261761A1, a biodegradable stent designed in particular for operations carried out within the gastrointestinal tract - in operations of intestinal anastomosis - is known. The stent has a special, slightly accordion-like crimped construction that ensures, at the same time, both the proper level of rigidity and elasticity.

In US patent application US2008208357A1, a stent used in case of damage of tissues of the gastrointestinal tract or adverse flow of fluids is disclosed. The stent has elongated round edgings on its edge, which can be coaxially extended outside the tubular space of the stent to facilitate anchoring of the stent.

A device according to the preamble of claim 1 is known from the US-A-2008/132989.

For introducing the above-mentioned stents to human body canals, catheters are used, i.e. systems, a structure of which depends mainly on whether the stent is self-expanding or is expandable by means of a balloon.

During introduction of self-expanding stents in a catheter, they remain retained in the contracted form in a shield-like external tube of the catheter. Then, the stent is released by an operator at the target site. It expands radially and takes a predefined shape, while at the same time exerting some pressure on body opening walls, in which it is being located. Stents of that type are usually made of a net of nitinol wires, which are not permanently joined at points of their crossing. Because of that, the stent in the contracted form may be located in a narrow shield-like tube and adopts a shape of a tube of smaller diameter and a longer length in relation to the target shape. On the other hand, upon expanding it adopts a target shape of a predefined greater diameter, and consequently, of a lower length (i.e. a stent that exhibits the "shape memory"). Expanding of the stent may be only slightly blocked by a size or unevenness of body cavities, in which the stent is being located. The stent must remain expanded and be able to remain at an installation site, i.e. it cannot undergo any migration or permanent deformation.

Stents expandable on a balloon, during their introducing remain in the contracted form outside the balloon. Then, the balloon is inflated to the desired shape, and along with it, the stent, which upon deflating the balloon is implanted at the target place. While inflating the balloon, the stent expands radially and fits with it shape to walls of body openings, in which it is being located. Pressure within a balloon presses the stent against walls, e.g. vessel walls. Systems and stents of that type are somewhat less suitable to be used in the gastrointestinal tract, e.g. in intestines, as they are more prone to deformation.

The main difficulty as regards location of stents in the gastrointestinal tract, in particular in intestines, is that - as compared to blood vessels - these organs exhibit more unevenness, have pockets or hollows, and are stimulated by the organism to various movements. Furthermore, if there are lesions within such an organ, their shape is even more complex and folded. In this situation, positioning the stent exactly in a narrowing or at a place of a complete obstruction of an intestine is even more complex.

Thus, there is a need to develop a system that makes it possible to position a stent precisely within body canals of such a type, as well as to develop a stent, which can be effectively implanted at the target position without a risk of displacement. In particular, the objects are achieved by a system according to claim 1. Preferred embodiments are subject of dependent claims.

System of the invention, for introducing and positioning stent, in particular an intestinal stent, comprising a distal part, a proximal part, an external tube and an internal tube, a canal of a guidewire, and a handle, is characterized in that in the distal part it is equipped with an entering tip and with an elastic expandable element, located behind the tip and fastened to the internal tube on its external surface, including inside at least one marker, visible in X-rays, and behind the elastic expandable element, around the internal tube, there is a stent located.

The elastic expandable element is a non-profiled balloon.

The stent is a self-expanding stent and from the side of the balloon is limited by a distal tip of an external tube that releases the stent.

If the balloon comprises a single marker inside, then the marker is located at the proximal part of the balloon, and when there are two markers, then the second, additional marker is located at the distal part of the balloon.

The stent is made of wires and is equipped with hooks constituting separate sections of the wires, fastened with one end to the stent by means of the clamp, comprising a wire built into the stent and one tip of the hook, whereas the second, free tip of the hook forms an angle with the stent surface, preferably it is perpendicular to the stent surface.

The stent is equipped with two to twelve hooks which preferably are fastened to the central part of the stent.

The stent is also equipped with markers which preferably are located at the both tips of the stent, at least one marker at each of the tips, and at least one is situated at the central part of the stent, a part of the markers or all the markers being at the same time clamps.

A self-expanding stent, in particular an intestinal stent, is made of wires and is equipped with hooks constituting separate sections of the wires, fastened with one end to the stent by means of the clamp comprising a wire built into the stent and one tip of the hook, whereas the second, free tip of the hook forms an angle with the stent surface, preferably it is perpendicular to the stent surface.

The stent is equipped with two to twelve hooks, which preferably are fastened to the central part of the stent.

The stent is equipped with markers which are preferably located at the both tips of the stent, at least one marker at each of the tips, and at least one marker is situated at the central part of the stent, a part of the markers or all the markers being at the same time clamps.

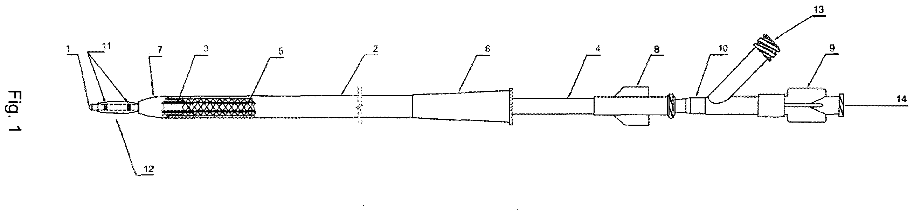

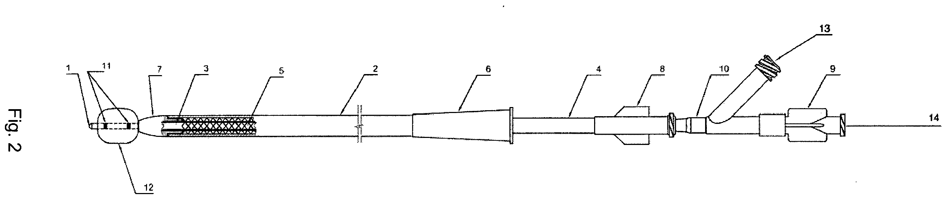

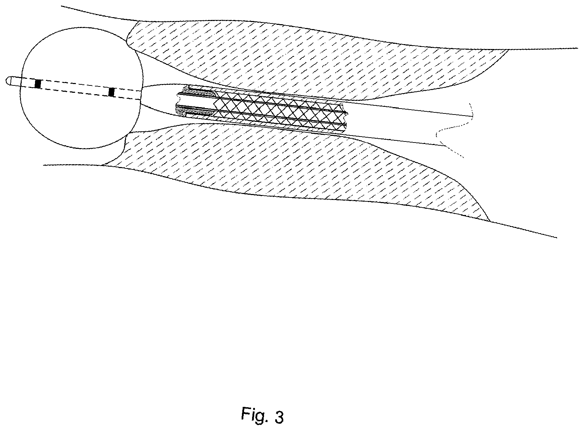

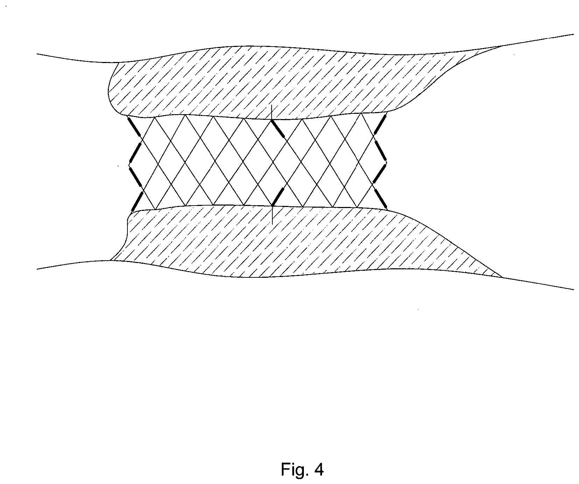

Object of the invention is shown in a representative embodiment on drawings, where fig. 1 shows a general view of the system with a deflated balloon, on fig.2 - the system with an inflated balloon, on fig.3 - an arrangement of the proximal part of the system during positioning of the stent in an intestine (magnified), on fig. 4 - a released stent upon withdrawing the system (magnified) - the stent is arranged in the target position along with hooks, placed within the tissue, positioned approximately perpendicular to the stent wall, and on fig 5. - the stent with hooks.

The system in its distal part (to be introduced as the first part during an operation) is equipped with a round entering tip 1 of the system, behind which there is a non-profiled, elastic balloon 12 connected with an internal tube 3. Behind the balloon, there is a distal tip 7 of the external tube 2 that liberates a self-expanding stent 5, the external tube 2 being ended with a handle 6, behind which there is ejector 4 ended with an attachment 8 being connected to an attachment 10. The attachment 10 includes a canal 13 for inflating and deflating the balloon, and it is connected with attachment 9 of a canal 14 beneath the guidewire. The canal 14 runs along the internal tube 3 and external tube 2. The canal 13, on the other hand, is connected with an attachment 10 to form askew an acute angle with an axis of the whole system. The balloon 12 is equipped with markers 11 present within the internal space of the balloon, and the balloon should have at least one marker in a part of the balloon neighboring to the stent. Preferably, there are two markers inside the balloon - one in the distal part, the second one - in the proximal part of the balloon, i.e. in the vicinity of the stent. The stent 5 is provided with markers 15 and hooks 16, located in its central part, that are fastened with clamps 17 to wires, from which the stent is made. The stent may include 2 to 20 markers, usually being arranged in three sections of the stent, i.e. at the two stent tips and in the central part of the stent. Preferably, the stent may include 1, 2, 3 or 4 markers at each stent tip and in the central part of the stent. The clamps located in the central part of the stent are at the same time, markers that label a position of the hooks.

The stent 15 is made of wires, e.g. nitinol wires and is equipped with hooks 16, concentrically arranged on its circumference. The stent includes at least two hooks. They are in the central part of stent. The stent may be provided with two, three or four hooks or with a greater number of hooks. They are made of separate wire sections, e.g. such from which a stent net is made. It can also be a section of wire having different thickness and different characteristics. Each hook is made in such a way that one free tip protrudes beyond a side wall of the stent and is bent to form a right or acute angle with side wall of the stent, whereas the second tip of the hook is fastened to a ring-shaped clamp, which at the same time can play a role of a marker. The hooks make it possible to place and position firmly the stent at narrowing or obstruction, where there is, e.g. the outgrown neoplastic tissue. The hooks prevent from displacing the stent. At releasing the stent during the operation, the hooks gradually embed into the outgrown tissue. Each clamp that fastens the hook at the same time may be a marker, but - when e.g. hooks are located on the circumference of the stent at the same distance from the stent edge - it is also possible that a single marker is sufficient, which signals a distance from the stent edge, on which the fastened hooks are located. If the stent placed in the system is in the contracted state, the hooks are pressed to the side wall of the stent. The stent can be within the system located in a position in which free tips of the hooks are arranged according to a direction of system movement during its introduction to body canals or contrary to this direction, i.e. the free tips of the hooks may be in the system arranged in such a way that they are directed to the distal part of the system or to the proximal part of the system. At releasing the stent, the hooks achieve primary, predefined shape - they come off the stent (they are bent in relation to the side surface of the stent).

It will be apparent for the person skilled in the art that the system is equipped with a canal for inflating and deflating the balloon, which has to be connected to further devices to ensure a typical procedure of inflating and deflating the balloon including a possibility of process control, e.g. control of pressure in the balloon. That part of the device does not constitute the subject matter of the invention and will be easy to anticipate basing on information known from literature of the art. For the skilled in the art, construction of a part of the system responsible for stent release, as both the method of releasing the self-expanding stent as well as that of the stent expanding on a balloon have been known for many years, will also be apparent.

The system for introducing and positioning stent of the invention is introduced into the body in such a way that it is directed - by means of the guidewire - to the target site, for example to an intestine, where a narrowing or the complete obstruction has been diagnosed. A narrow entering tip 1 is pushed through such that the narrowing (or the obstruction) along with a non-inflated balloon 12. Pushing-through the balloon 12 may be observed by means of markers 11 located inside the balloon. In this location the balloon becomes inflated in such a way that its return slipping-out through the narrowing area is not possible, yet it is possible maximum retracting of the balloon, to support the balloon on edges of the narrowing, which causes that the external tube 2 that releases the self-expanding stent 5 will be positioned exactly at the narrowing site. The balloon may be designed to be inflated with contrast liquid, which makes it possible to set precisely the balloon position, and thus that of the system, and optionally to correct balloon position through tightening it to narrowing walls by means of a slight withdrawing of the system with the inflated balloon. Once the desired system position is achieved with a use of the ejector 4, the self-expanding stent is being released. The stent position may be monitored with a use of markers 15. The open (i.e. expanded) stent widens a lumen of intestine, thus eliminating the narrowing.

The system of the invention makes it possible to locate precisely the stent exactly at a narrowing place in the gastrointestinal tract or in an intestine. An elastic balloon is non-profiled, which means that its shape was not specifically programmed, e.g. to be a cylinder of a definite diameter. If there is no hindrance during inflating, it expands uniformly radially and will not adopt any predefined shape, or even it has not to expand symmetrically, but it will fit to unevenness of a surrounding overgrown tissue that causes body canal narrowing. The balloon can - on one hand - expand considerably, and - on the other hand - may be blocked by a non-symmetrically overgrown tissue. Balloon elasticity has been ensured through a use of special materials. These can be e.g. polyurethane, latex or elastomer-based materials.

Such construction of a balloon prevents from damage of surrounding tissue, as the balloon is soft and easily accommodates to folding of a body organ. The presented construction of the balloon makes it possible to support it on edges of an overgrown tissue near a site of maximum canal lumen narrowing, to which the system is introduced. Behind the balloon, at a site of maximum tissue narrowing, the stent is then positioned, which is precisely released at the site of intestine lumen narrowing.

The system for introducing and positioning stent, in particular intestinal stent, may also be a system that includes a stent expanding on a balloon. It will be apparent for the skilled in the art how to adopt the construction described above to a system with a stent released through expanding on a balloon. Such a system does not form part of the invention.

On the other hand, the system with a self-expanding stent seems to be better in case of obstructions of the gastrointestinal tract, in particular intestines. Self-expanding stents permanently press on walls of contracted intestine, are more resistant to deformation, more elastic and stable, and maintain its shape during functioning of movable tissues.

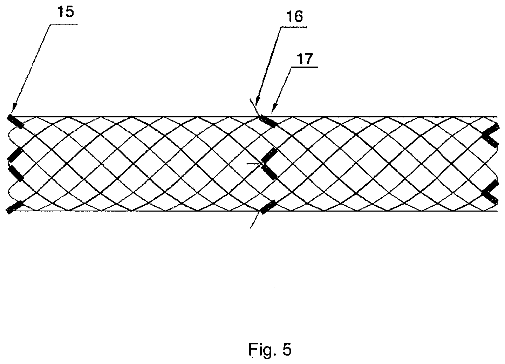

Self-expanding stent shown on fig. 5 is equipped with hooks, which upon stent releasing are being stuck into tissue and thanks to it the stent is permanently positioned. Through its constant expanding force exerting on body canal walls, it presses onto the hooks, which hold the stent in the desired position.

### Designations on drawings:

- 1.: Entering tip
- 2.: External tube releasing the stent
- 3.: Internal tube
- 4.: Ejector
- 5.: Self-expanding stent
- 6.: Handle
- 7.: Distal tip of external tube releasing the stent
- 8.: Ejector attachment
- 9.: Attachment of a guidewire canal
- 10.: Attachment of a canal for inflating and deflating the balloon
- 11.: Balloon markers
- 12.: Balloon
- 13.: Canal for inflating and deflating the balloon
- 14.: Canal for a guidewire
- 15.: Stent marker
- 16.: Hook
- 17.: Clamp of the hook

## Claims

1. A system for introducing and positioning a stent, in particular an intestinal stent, comprising a distal part, a proximal part, an external tube (2) releasing a stent and an internal tube (3), canal of a guidewire (14) and a handle (6), the system is equipped with an entering tip (1) positioned in the distal part and with an elastic expandable element (12) located behind the entering tip (1) and fastened to the internal tube (3) on its external surface, a self-expanding stent (5) located behind the elastic expandable element formed by a non-profiled balloon (12), and around the internal tube (3), **characterised in that** the stent (5) is limited from the side of the balloon (12) by a distal tip (7) of the external tube (2) releasing the stent (5), and wherein at least one balloon marker (11), that is visible in X-rays, is present within the internal space of the balloon (12).

2. The system according to Claim 1, **characterized in that** when the balloon (12) includes inside one marker (11), then the marker (11) is located in the proximal part of the balloon at the vicinity of the stent (5), and if the balloon (12) includes inside two markers (11), then the second, additional marker, is located in the distal part of the balloon.

3. The system according to Claim 1 or 2, **characterized in that**, the stent (5) is made of wires and is equipped with hooks (16) constituting separate sections of the wires, fastened with one end to the stent by means of the clamp (17), comprising a wire built into the stent and one tip of the hook, whereas the second, free tip of the hook forms an angle with the stent surface, and preferably the free tip of the hook is perpendicular to the stent surface.

4. The system according to Claim 3, **characterized in that** the stent (5) is equipped with two to twelve hooks (16), which preferably are fastened to the central part of the stent.

5. The system according to Claim 3 or 4, **characterized in that** the stent (5) is equipped with markers (15), which preferably are located at the both tips of the stent, at least one at each of the tips, and at least one is situated at the central part of the stent, whereas a part of the markers (15) or all the markers (15) being at the same time the clamps (17).

## Patentansprüche

1. Ein System zum Einführen und Positionieren eines Stents, insbesondere eines intestinalen Stents, umfassend einen distalen Teil, einen proximalen Teil, ein äußeres Rohr (2), das einen Stent freigibt, und ein inneres Rohr (3), einen Kanal für einen Führungsdraht (14) und einen Griff (6), wobei das System mit einer Eintrittsspitze (1), die im distalen Teil positioniert ist, und mit einem elastischen dehnbaren Element (12) ausgestattet ist, das hinter der Eintrittsspitze (1) angebracht und an der Außenfläche des inneren Rohrs (3) befestigt ist, einen selbst ausdehnbaren Stent (5), der hinter dem elastischen ausdehnbaren Element, das durch einen nicht profilierten Ballon (12) gebildet wird, und um die innere Röhre (3) herum angebracht ist, **dadurch gekennzeichnet, dass** der Stent (5) von der Seite des Ballons (12) durch eine distale Spitze (7) der äußeren Röhre (2) begrenzt wird, die den Stent (5) freigibt, und wobei mindestens eine Ballonmarkierung (11), die in Röntgenstrahlen sichtbar ist, im Innenraum des Ballons (12) vorhanden ist.

2. Das System nach Anspruch 1, **dadurch gekennzeichnet, dass** wenn der Ballon (12) im Inneren eine Markierung (11) aufweist, dann befindet sich die Markierung (11) im proximalen Teil des Ballons in der Nähe des Stents (5), und wenn der Ballon (12) im Inneren zwei Markierungen (11) aufweist, dann befindet sich die zweite, zusätzliche Markierung im distalen Teil des Ballons.

3. Das System nach Anspruch 1 oder 2, **dadurch gekennzeichnet, dass** der Stent (5) aus Drähten besteht und mit Haken (16) versehen ist, die getrennte Abschnitte der Drähte bilden und mit einem Ende mittels der Klemme (17) am Stent befestigt sind, die einen in den Stent eingebauten Draht und eine Hakenspitze umfasst, während die zweite, freie Hakenspitze mit der Stentfläche einen Winkel bildet und vorzugsweise die freie Hakenspitze senkrecht zur Stentfläche steht.

4. Das System nach Anspruch 3, **dadurch gekennzeichnet, dass** der Stent (5) mit zwei bis zwölf Haken (16) ausgestattet ist, die vorzugsweise am zentralen Teil des Stents befestigt sind.

5. Das System nach Anspruch 3 oder 4, **dadurch gekennzeichnet, dass** der Stent (5) mit Markern (15) versehen ist, die sich vorzugsweise an beiden Spitzen des Stents befinden, wobei sich mindestens einer an jeder der Spitzen und mindestens einer im zentralen Teil des Stents befindet, während ein Teil der Marker (15) oder alle Marker (15) gleichzeitig die Klemmen (17) sind.

## Revendications

1. Un système pour introduire et positionner une endoprothèse, en particulier une endoprothèse intestinale, comprenant une partie distale, une partie proximale, un tube externe (2) libérant une endoprothèse et un tube interne (3), un canal d'un fil de guidage (14) et un poignée (6), le système est équipé d'un embout d'entrée (1) positionné dans la partie distale et d'un élément extensible élastique (12) situé derrière l'embout d'entrée (1) et fixé au tube interne (3) sur sa surface externe, une endoprothèse auto-extensible (5) située derrière l'élément extensible élastique formé par un ballon non profilé (12), et autour du tube interne (3), **caractérisé en ce que** l'endoprothèse (5) est limitée du côté du ballon (12) par un embout distal (7) du tube externe (2) libérant l'endoprothèse (5), et dans lequel au moins un marqueur de ballon (11), qui est visible dans les rayons X, est présent dans l'espace interne du ballon (12).

2. Le système selon la revendication 1, **caractérisé en ce que** lorsque le ballon (12) comprend à l'intérieur un marqueur (11), alors le marqueur (11) est situé dans la partie proximale du ballon au voisinage de l'endoprothèse (5), et si le ballon (12) comprend à l'intérieur deux marqueurs (11), alors le second marqueur supplémentaire est situé dans la partie distale du ballon.

3. Le système selon la revendication 1 ou 2, **caractérisé en ce que** l'endoprothèse (5) est constitué de fils et est équipé de crochets (16) constituant des sections séparées des fils, fixées avec une extrémité à l'endoprothèse au moyen de la pince (17), comprenant un fil intégré dans l'endoprothèse et un embout du crochet, tandis que le second embout libre du crochet forme un angle avec la surface de l'endoprothèse, et de préférence l'embout libre du crochet est perpendiculaire à la surface de l'endoprothèse.

4. Le système selon la revendication 3, **caractérisé en ce que** l'endoprothèse (5) est équipé de deux à douze crochets (16), qui sont de préférence fixés à la partie centrale de l'endoprothèse.

5. Le système selon la revendication 3 ou 4, **caractérisé en ce que** l'endoprothèse (5) est équipée de marqueurs (15), qui sont de préférence situés aux deux embouts de l'endoprothèse, au moins un à chacun des embouts, et au moins un est situé à la partie centrale de l'endoprothèse, alors qu'une partie des marqueurs (15) ou tous les marqueurs (15) sont en même temps les pinces (17).
